# EUROPEAN PATENT SPECIFICATION

(11) **EP 3 831 312 B1**
(45) Date of publication and mention of the grant of the patent: **18.02.2026**
(21) Application number: 19911167.5
(22) Date of filing: 14.11.2019
(51) Int. Cl.: A61B 17/072

(54) **SURGICAL INSTRUMENT AND LINEAR STAPLER**
CHIRURGISCHES INSTRUMENT UND LINEARES KLAMMERGERÄT
INSTRUMENT CHIRURGICAL ET AGRAFEUSE LINÉAIRE

(30) Priority: 24.01.2019 CN 201910067871
(43) Date of publication of application: 09.06.2021
(73) Proprietor: IntoCare Medical Technology (Suzhou) Co., Ltd., Suzhou, Jiangsu 215000 (CN)
(72) Inventor: XU, Ronghua, Suzhou, Jiangsu 215000 (CN); ZHANG, Hui, Suzhou, Jiangsu 215000 (CN); DU, Yunfeng, Suzhou, Jiangsu 215000 (CN); LIU, Dianchen, Suzhou, Jiangsu 215000 (CN)
(74) Representative: Gulde & Partner
(86) International application number: PCT/CN2019/118394
(87) International publication number: WO 2020/151335

(56) References cited:
- EP-A1- 3 225 177
- CN-A- 104 287 800
- CN-A- 104 434 244
- CN-A- 105 476 678
- CN-A- 107 928 733
- CN-B- 106 388 893
- CN-U- 202 526 241
- CN-U- 206 809 304
- US-A- 3 692 224
- US-A1- 2002 084 304
- US-A1- 2017 281 155
- US-B2- 8 955 732

## Description

### TECHNICAL FIELD

The present disclosure relates to a surgical instrument and a linear surgical stapler, and belongs to the field of medical instruments.

### BACKGROUND

A linear surgical stapler, also known as a digestive tract closer, is an instrument that insert a plurality of staples staggered each other into a target tissue in a straight line. The linear surgical stapler is one of the instruments widely used to replace a linearly manual suturing, is mainly used to suture and close the stumps of stomach, duodenum, small intestine, and colon and is a full-layer eversion suturing instrument.

A manner of inserting the staples into the target tissue by the linear surgical stapler in the prior art is usually that a transmission device pushes an anvil to push out and fire all of the staples at one time. In this case, the staples are prone to having large and small heads, a dovetail and the like so that an inserting effect is poor, resulting in a poor suturing effect and waste of staples. In addition, because a large number of staples are fired at one time, a part of the staples cannot be firmly inserted into the target tissue, resulting in tissue leakage. In addition, because a large number of staples need to be fired at one time, a relatively large firing force is needed, which causes the anvil with a cantilever beam structure to deform after a plurality of firings.

US2002/0084304A1 discloses a stapling device for use as an attachment to an electromechanical device driver comprises an upper jaw and a lower jaw, the upper jaw having staple guides corresponding to one or more staples in a removable staple tray disposed within a lower jaw, whereby a wedge having a threaded bore travels upon a matching threaded shaft in a channel disposed in the lower jaw behind the staple tray, such that rotation of the threaded shaft causes movement of the wedge through the channel while a sloped surface of the wedge contacts the staples to push the staples against the staples guides, closing the staples.

### SUMMARY

It is an objective of the present disclosure to provide a surgical instrument and a linear surgical stapler, which fire the staples sequentially, and thus can reduce the firing force to avoid the deformation of the anvil, and have a better insertion effect of the staples into the tissue, a better suturing effect and surgical safety.

The objective can be achieved by respective independent claims. Further embodiments of the present invention are defined in the corresponding dependent claims.

### BRIEF DESCRIPTION OF THE DRAWINGS

FIG. 1 is a schematic diagram of an external structure of a surgical instrument according to embodiments of the present disclosure;
FIG. 2 is a schematic diagram of an overall structure of the surgical instrument according to the embodiments of the present disclosure;
FIG. 3 is a schematic diagram of a distal end of the surgical instrument according to the embodiments of the present disclosure;
FIG. 4 is a schematic structural diagram of a transmission assembly of the surgical instrument according to the embodiments of the present disclosure;
FIG. 5 is a schematic structural diagram of a proximal end of the surgical instrument according to the embodiments of the present disclosure;
FIG. 6 is a schematic structural diagram of a linear stapler in an initial state according to the embodiments of the present disclosure;
FIG. 7 is a schematic structural diagram of the linear stapler in a closed state according to the embodiments of the present disclosure;
FIG. 8 is a schematic structural diagram of the linear stapler in a firing state according to the embodiments of the present disclosure.

### DETAILED DESCRIPTION

The exemplary specific implemented modes of the present disclosure will be described in further detail below in conjunction with the drawings and embodiments. The following embodiments are intended to illustrate the present disclosure, but not to limit the scope of the present disclosure.

In the description of the present disclosure, it should be noted that the orientation or positional relationship indicated by the terms such as "center", "upper", "lower", "left", "right", "front", "rear", "axis direction", "inner", and "outer" are based on the orientation or positional relationship shown in the drawings, which are merely intended for describing the present disclosure and simplifying the description, but do not indicate or imply that the concerned device or element shall have a specific orientation, or be constructed and operated in a specific orientation, so that these terms cannot be understood as a limitation to the present disclosure. In addition, the terms such as "first", "second", and "third" are merely used for descriptive purposes, and cannot be understood as indicating or implying relative importance.

In the description of the present disclosure, it should be noted that the terms such as "mounting", "connecting" and "connection" should be understood in a general sense, unless otherwise clearly specified and limited. For example, these terms may refer to a fixed connection, a detachable connection or an integral connection, a mechanical connection or an electrical connection, a direct connection or an indirect connection through an intermediate medium, or an internal communication between two elements. For a person of ordinary skill in the art, the specific meanings of the foregoing terms in the present disclosure may be understood according to specific situations.

In addition, the technical features involved in different embodiments of the present disclosure described below may be combined with each other as long as there is no conflict therebetween.

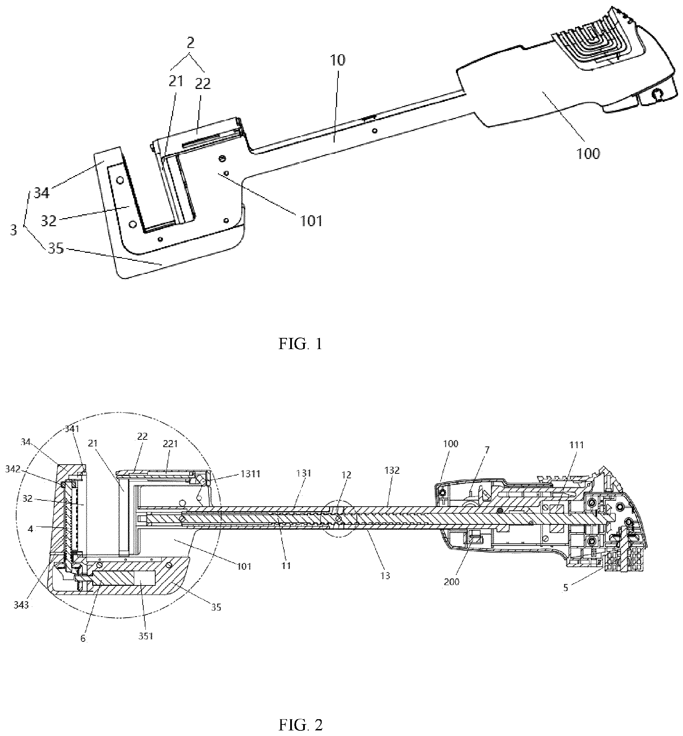

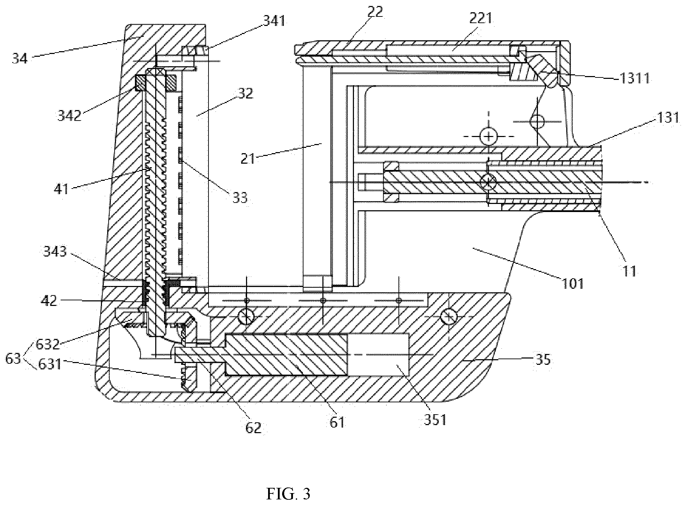

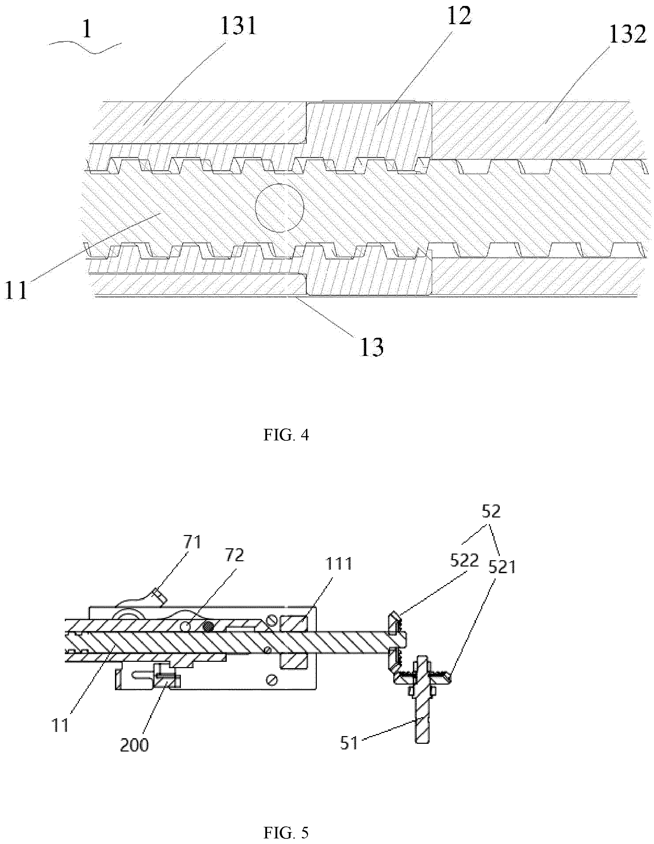

Referring to FIGS 1 to 5, a surgical instrument according to embodiments of the present disclosure includes: a transmission assembly 1, in which the transmission assembly 1 is provided with a mounting part 101 for mounting a suturing mechanism, the suturing mechanism (i.e. an end effector) includes an anvil assembly 2 and a staple cartridge assembly 3, the staple cartridge assembly 3 includes a staple cartridge 32 for accommodating staples 31, the anvil assembly 2 includes an anvil 21 matched with the staple cartridge 32, and the transmission assembly 1 is connected to the anvil assembly 2 and drives the anvil assembly 2 to move relative to the staple cartridge assembly 3; a staple pushing assembly 4 configured to push the staples 31 to move toward the anvil assembly 2, in which the staple pushing assembly 4 includes a first threaded rod 41 provided on the staple cartridge assembly 3 and a firing nut 42 sleeved on the first threaded rod 41, and an axis direction of the first threaded rod 41 and the firing nut 42 is defined as a first axis direction (a straight line of Arrow A in FIG. 2); a drive assembly 5 connected to the transmission assembly 1 to drive the transmission assembly 1 to move. The surgical instrument of the embodiments of the present disclosure further includes a firing assembly 6 provided on the anvil assembly 2. The firing assembly 6 is connected to the first threaded rod 41 and drives the first threaded rod 41 to rotate, so that the firing nut 42 moves relative to the first threaded rod 41 in the first axis direction.

In the embodiments of the present disclosure, the surgical instrument includes two plate pieces 10 as a main body of the surgical instrument. The transmission assembly 1 is mechanically connected with the plate pieces 10. The suturing mechanism is provided at a distal end of the plate pieces 10 (in a direction indicated by Arrow B in FIG. 2). For example, the distal end of the plate pieces 10 is provided with the mounting part 101. The mounting part 101 is a concave structure. The anvil 21 is provided on a right side of the concave structure. The staple cartridge 32 is mounted on a left side of the concave structure. A staple pushing sheet 33 for accommodating and firing the staples 31 is further provided in the staple cartridge 32. The structure of the staple pushing sheet 33 may refer to common design, which is not detailed here. The anvil assembly 2 further includes a connecting part 22. The connecting part 22 connects the anvil 21 and the mounting part 101. An upper tissue positioning needle 221 is provided in the connecting part 22. An upper tissue positioning hole 341 corresponding to the upper tissue positioning needle 221 is provided on the staple cartridge assembly 3. If the suturing mechanism is closed, the upper tissue positioning needle 221 is inserted into the upper tissue positioning hole 341 to fix the tissue to be sutured. The staple cartridge assembly 3 further includes an upper jaw arm 34 and a lower jaw arm 35. The upper jaw arm 34 is provided at an outer side of the staple cartridge 32 to protect the staple cartridge 32. The upper jaw arm 34 is provided with a slot hole (not numbered) for accommodating the first threaded rod 41 and the firing nut 42. A first bearing 342 for fixing the first threaded rod 41 is provided in the slot hole. The lower jaw arm 35 is provided with a hollow chamber 351 for accommodating the firing assembly, and the firing assembly 6 is mechanically fixed in the hollow chamber 351. In the embodiments of the present disclosure, the upper tissue positioning hole 341 is provided on the upper jaw arm 34, and a lower tissue positioning needle 343 is fixed on the upper jaw arm 34. The lower tissue positioning needle 343 is inserted into the anvil assembly 2 if the suturing mechanism is closed.

In the embodiments of the present disclosure, the transmission assembly 1 includes a transmission rod 11. The transmission rod 11 is connected to the drive assembly 5 and rotates under the action of the drive assembly 5. An axis direction of the transmission rod 11 is defined as a second axis direction (a straight line of Arrow B in Figure 2). The first axis direction is perpendicular to the second axis direction. In the embodiments of the present disclosure, the transmission rod 11 is provided between the two plate pieces 10. The transmission rod 11 is a second threaded rod. A threaded sleeve 12 is sleeved on the second threaded rod 11. The threaded sleeve 12 is preferably provided at a middle portion of the second threaded rod 11. The drive assembly 5 drives the second threaded rod 11 to rotate, then the threaded sleeve 12 moves relative to the second threaded rod 11 in the second axis direction. In the embodiments of the present disclosure, the second threaded rod 11 for example is provided with threads only at the middle portion of the second threaded rod 11. If the suturing mechanism is closed, the threaded sleeve 12 is located at a distal end of the threads of the second threaded rod 11.

In the embodiments of the present disclosure, the firing assembly 6 includes a firing motor 61, a firing input shaft 62 provided on the firing motor 61, and a direction-changing transmission device 63 respectively connected to the firing input shaft 62 and the first threaded rod 41. After the suturing mechanism is closed, the firing motor 61 drives the first threaded rod 41 to rotate by the direction-changing transmission device 63. For example, the direction-changing transmission device 63 includes a first bevel gear 631 sleeved on the firing input shaft 62 and a second bevel gear 632 sleeved on the first threaded rod 41. The first bevel gear 631 and the second bevel gear 632 are engaged with each other to transform a rotation of the firing input shaft 62 with respect to the second axis direction into a rotation of the first threaded rod 41 with respect to the first axis direction. Of course, in other embodiments, the direction-changing transmission device 63 may have other structures, as long as the direction-changing transmission is achieved.

In the embodiments of the present disclosure, the transmission assembly 1 further includes a closure assembly 13 sleeved on the second threaded rod 11. The closure assembly 13 includes a first closure rod 131 and a second closure rod 132. The threaded sleeve 12 is fixedly connected to the first closure rod 131 and the second closure rod 132. The threaded sleeve 12 is provided between the first closure rod 131 and the second closure rod 132. For example, the first closure rod 131 is connected to the connecting part 22 of the anvil assembly 2, and the second closure rod 132 is provided closer to the drive assembly 5 than the first closure rod 131 (i.e. the second closure rod 132 is at a proximal end). The drive assembly 5 drives the second threaded rod 11 to rotate, the threaded sleeve 12 moves relative to the second threaded rod 11 in the second axis direction, thereby driving the first closure rod 131 and the second closure rod 132 to move in the second axis direction. The first closure rod 131 is connected to the anvil assembly 2, so that during the threaded sleeve 12 moves toward the distal end of the second threaded rod 11, the first closure rod 131 pushes the anvil assembly 2 to move toward the staple cartridge assembly 3 to close the suturing mechanism. The first closure rod 131 and the second closure rod 132 are integral with each other, or are provided separately and then connected together. Preferably, the first closure rod 131 and the second closure rod 132 are integral with each other, and are provided with a slot hole (not shown) for receiving the threaded sleeve 12.

In the embodiments of the present disclosure, a protruding rod 1311 is provided on the first closure rod 131. The protruding rod 1311 abuts against a proximal end of the upper tissue positioning needle 221. During the first closure rod 131 drives the anvil assembly 2 to move toward the staple cartridge assembly 3, the protruding rod 1311 drives the upper tissue positioning needle 221 to move toward the upper tissue positioning hole 341 and insert into the upper tissue positioning hole 341, so that the tissue to be sutured is positioned by the upper tissue positioning needle 221 so as to facilitate subsequent suturing and cutting.

The surgical instrument of the embodiments of the present disclosure further includes a safety switch 7 serving as a stopper. The safety switch 7 is provided on the closure assembly 13. For example, the safety switch 7 includes a switching knob 71 and a switching shaft (not shown). The switching shaft is connected to the switching knob 71. The second closure rod 132 is provided with a through hole 72 adapted to the switching shaft. After the suturing mechanism is closed, the switching knob 71 is toggled to insert the switching shaft into the through hole 72 to lock the closure assembly 13. The safety switch 7 may refer to common design, which is not detailed here.

The surgical instrument of the present disclosure further includes a handle 100. The transmission rod 11 is fixed to the handle 100 by a second bearing 111. The second closure rod 132 and the drive assembly 5 are fixed to the handle 100. A manner of fixing may refer to common manner, such as a thread connection manner, a bonding manner, or a snap connection manner, which is not limited herein. The drive assembly 5 includes a drive motor (not shown), an input shaft 51 connected to the drive motor, and a transmission member 52 respectively connected to the input shaft 51 and the transmission rod 11. The transmission member 52 includes a first bevel gear 521 sleeved on the input shaft 51 and a second bevel gear 522 sleeved on the transmission rod 11. The first bevel gear 521 is engaged with the second bevel gear 522 so as to transform a rotation of the input shaft 51 with respect to the first axis direction into a rotation of the transmission rod 11 with respect to the second axis direction.

In the embodiments of the present disclosure, the surgical instrument further includes an induction switch 200 for sensing a position of the staple cartridge assembly 3. The induction switch 200 is fixed in the handle 100; and a manner of fixing may refer to common design, such as the thread connection manner, the bonding manner, or the snap connection manner, which is not limited herein. The induction switch 200 is a Hall switch or a photoelectric switch. After the suturing mechanism is closed and the closure assembly 13 is stationary, the staple cartridge assembly 3 at this time is provided at a position that the staple cartridge 32 and the anvil 21 are closed together, and the position of the staple cartridge assembly 2 is acquired by the induction switch 200.

In the embodiments of the present disclosure, a plugging hole for external power (not shown), a step indicator (not shown), and an operation button (not shown) are also provided on the handle 100. The plugging hole for example is connected to a power line of a medical power supply. Of course, the instrument may be powered by a built-in power supply, such as a battery.

Preferably, the surgical instrument of the embodiments of the present disclosure is a linear surgical stapler. The suturing mechanism is mounted on the mounting part 101. The suturing mechanism includes the anvil assembly 2 and the staple cartridge assembly 3 as described above.

The linear surgical stapler operates as follows.

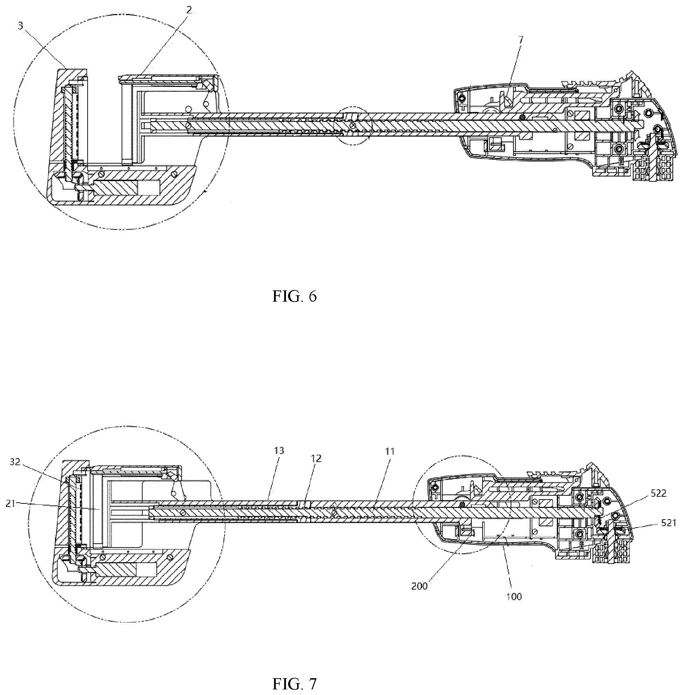

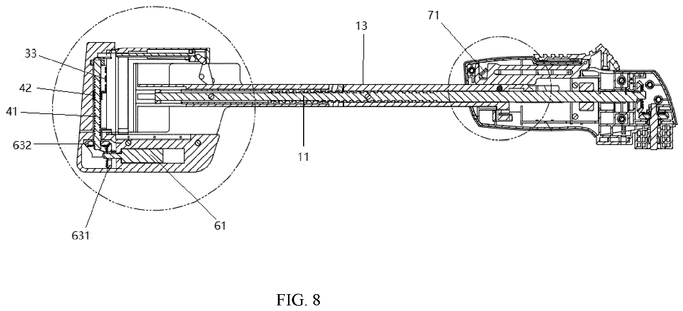

Referring to FIG. 6, in an initial state, the anvil assembly 2 is spaced from the staple cartridge assembly 3, and at this time, the safety switch 7 is in an "open" state at this time. Referring to FIG. 7, the operating button on the handle 100 is pressed, and the drive motor drives the input shaft 51 to rotate; due to the engagement between the first bevel gear 521 and the second bevel gear 522, the second threaded rod 11 is driven to rotate, the threaded sleeve 12 sleeved on the second threaded rod 11 is driven to push the closure assembly 13 to move toward the distal end of the second threaded rod 11 until the target tissue is clamped by the anvil assembly 2 and the staple cartridge assembly 3, the suturing mechanism is in the closed state (i.e. a state in which the target tissue is clamped by the anvil assembly 2 and the staple cartridge assembly 3), and the closure assembly 13 is stationary; at this time, the staple cartridge 32 is at the position that the staple cartridge 32 and the anvil 21 are closed together, the position of the staple cartridge 32 is acquired by the induction switch 200 (such as the photoelectric switch). Referring to FIG. 8, after the suturing mechanism is in the closed state, the switching knob 71 is toggled, and the safety switch 7 is in a "locked" state; at this time, the closure assembly 13 is locked; then, the firing motor 61 is driven to rotate, the first bevel gear 631 and the second bevel gear 632 are engaged with each other to drive the first threaded rod 41 to rotate, so that the first threaded rod 41 drives the firing nut 42 to move upward in the first axis direction, thereby pushing the staple pushing sheet 33 to sequentially fire the staples. The firing nut 42 automatically returns to an initial position after the firing is completed. After the firing of the staples is completed, the firing nut 42 automatically returns to the initial position. After the firing of the staples is completed, the safety switch is turned back to the "open" state, the drive motor rotate reversely, the second threaded rod 11 rotate reversely to drive the closure assembly 13 to move back so that a distance between the staple cartridge assembly 3 and anvil assembly 2 restores to the maximum (which may be controlled by the number of turns of the drive motor), which is consistent with the initial state.

In summary, the surgical instrument and the linear surgical stapler of the present disclosure are provided with the staple pushing assembly and the firing assembly. If the suturing mechanism is closed, the firing assembly drives the first threaded rod of the staple pushing assembly to rotate so as to drive the firing nut sleeved on the first threaded rod to move relative to the first threaded rod in the first axis direction, so that the staples in the staple cartridge are fired sequentially. Therefore, there is no problem such as large and small heads, dovetail and the like. In addition, the surgical instrument and the linear surgical stapler fire the staples sequentially, which can reduce the firing force to avoid the deformation of the anvil, and have a better insertion effect of the staples into the tissue, a better suturing effect and surgical safety.

The technical features of the foregoing embodiments may be combined arbitrarily. In order to make the description concise, all possible combinations of the various technical features in the foregoing embodiments are not described. However, as long as there is no conflict in the combination of these technical features, all combinations should be considered as within the scope of the disclosure.

The foregoing embodiments merely are some of the embodiments of the present disclosure and the descriptions of the foregoing embodiments are specific and detailed, but the foregoing embodiments should not be understood as limiting of the scope of the disclosure. It should be pointed out that a person of ordinary skill in the art may make modifications and improvements without departing from the concept of the present disclosure, and all of these modifications and improvements belong to the protection scope of the present disclosure. Therefore, the protection scope of the present disclosure should be defined by the appended claims.

## Claims

1. A surgical instrument, comprising:
a suturing mechanism comprising an anvil assembly (2) and a staple cartridge assembly (3),
a transmission assembly (1), wherein the transmission assembly (1) is provided with a mounting part (101), the suturing mechanism is mounted on the mounting part (101), the staple cartridge assembly (3) comprises a staple cartridge (32) for accommodating staples (31), the anvil assembly (2) comprises an anvil (21) matched with the staple cartridge (32), and the transmission assembly (1) is connected to the anvil assembly (2) and drives the anvil assembly (2) to move relative to the staple cartridge assembly (3);
a staple pushing assembly (4) configured for pushing the staples (31) to move toward the anvil assembly (2), wherein the staple pushing assembly (4) comprises a first threaded rod (41) provided on the staple cartridge assembly (3) and a firing nut (42) sleeved on the first threaded rod (41), an axis direction of the first threaded rod (41) and the firing nut (42) is defined as a first axis direction; and
a drive assembly (5), connected to the transmission assembly (1) to drive the transmission assembly (1) to move; wherein
the surgical instrument further comprises a firing assembly (6) provided on the anvil assembly (2), the firing assembly (6) is connected to the first threaded rod (41) and drives the first threaded rod (41) to rotate, so that the firing nut (42) moves relative to the first threaded rod (41) in the first axis direction; **characterized in that**,
the firing assembly (6) comprises a firing motor (61), a firing input shaft (62) provided on the firing motor (61), and a direction-changing transmission device (63) respectively connected to and between the firing input shaft (62) and the first threaded rod (41); and
if the suturing mechanism is closed, the firing motor (61) drives the first threaded rod (41) to rotate by the direction-changing transmission device (63);
wherein the transmission assembly (1) comprises a transmission rod (11), one end of the transmission rod (11) is connected to the drive assembly (5) and rotates under an action of the drive assembly (5), and the other end of the transmission rod (11) is connected to an anvil (21) included in the anvil assembly (2), an axis direction of the transmission rod (11) is defined as a second axis direction, and the first axis direction is perpendicular to the second axis direction.

2. The surgical instrument according to claim 1, wherein the direction-changing transmission device (63) comprises a first bevel gear (631) sleeved on the firing input shaft (62) and a second bevel gear (632) sleeved on the first threaded rod (41), and the first bevel gear (631) and the second bevel gear (632) are engaged with each other.

3. The surgical instrument according to claim 2, wherein
the transmission rod (11) is a second threaded rod (11), and a threaded sleeve (12) is sleeved on the second threaded rod (11);
the drive assembly (5) drives the second threaded rod (11) to rotate, so that the threaded sleeve (12) moves relative to the second threaded rod (11) in the second axis direction.

4. The surgical instrument according to claim 3, wherein
the transmission assembly (1) further comprises a closure assembly (13) sleeved on the second threaded rod (11), the closure assembly (13) comprises a first closure rod (131) and a second closure rod (132), the threaded sleeve (12) is fixedly connected to the first closure rod (131) and the second closure rod (132), the threaded sleeve (12) is provided between the first closure rod (131) and the second closure rod (132);
the first closure rod (131) is connected to the anvil assembly (2), the second closure rod (132) is provided closer to the drive assembly (5) than the first closure rod (131);
if the drive assembly (5) drives the second threaded rod (11) to rotate, the threaded sleeve (12) moves relative to the second threaded rod (11) in the second axis direction, thereby driving the first closure rod (131) and the second closure rod (132) to move in the second axis direction.

5. The surgical instrument according to claim 4, wherein
the surgical instrument further comprises a safety switch (7) serving as a stopper, the safety switch (7) is provided on the closure assembly (13), the safety switch (7) comprises a switching knob (71) and a switching shaft, the switching shaft is connected to the switching knob (71), and the second closure rod (132) is provided with a through hole (72) adapted to the switching shaft; and
if the suturing mechanism is closed, the switching knob (71) is toggled, the switching shaft is inserted into the through hole (72) to lock the closure assembly (13)..

6. The surgical instrument according to claim 1, wherein
the anvil assembly further comprises an upper jaw arm (34) and a lower jaw arm (35), the upper jaw arm (34) is provided at an outer side of the staple cartridge (32), the upper jaw arm (34) is provided with a slot hole for accommodating the first threaded rod and the firing nut, a first bearing for fixing the first threaded rod is provided in the slot hole;
the lower jaw arm (35) is provided with a hollow chamber (351) for accommodating the firing motor (61) and the firing input shaft (62) of the firing assembly (6), and the firing assembly (6) is mechanically fixed in the hollow chamber (351).

7. The surgical instrument according to claim 1, wherein the surgical instrument further comprises a handle (100), the transmission rod (11) is fixed to the handle (100) by a second bearing, the drive assembly (5) is provided in the handle (100), and the drive assembly (5) comprises a drive motor, an input shaft (51) connected to the drive motor, and a transmission member (52) respectively connected to the input shaft (51) and the transmission rod (11).

8. The surgical instrument according to claim 7, wherein the surgical instrument further comprises an induction switch (200) for sensing a position of the staple cartridge assembly (3), and the induction switch (200) is provided in the handle (100).

9. The surgical instrument according to claim 1, further comprising a linear surgical stapler.

10. The surgical instrument according to claim 3, wherein
the second threaded rod (11) is provided with threads only at a middle portion of the second threaded rod (11); and
if the suturing mechanism is closed, the threaded sleeve (12) is located at a distal end of the threads of the second threaded rod (11).

11. The surgical instrument according to claim 6, wherein a lower tissue positioning needle (343) is fixed on the upper jaw arm (34), and the lower tissue positioning needle (343) is inserted into the anvil assembly (2) if the suturing mechanism is closed.

12. The surgical instrument according to claim 7, wherein the transmission member (52) includes a first bevel gear (521) sleeved on the input shaft (51) and a second bevel gear (522) sleeved on the transmission rod (11), the first bevel gear (521) is engaged with the second bevel gear (522 so as to transform a rotation of the input shaft (51) with respect to the first axis direction into a rotation of the transmission rod (11) with respect to the second axis direction.

## Patentansprüche

1. Chirurgisches Instrument, umfassend:
einen Nahtmechanismus, der eine Ambossbaugruppe (2) und eine Klammermagazinbaugruppe (3) umfasst,
eine Übertragungsbaugruppe (1), wobei die Übertragungsbaugruppe (1) mit einem Montageteil (101) versehen ist, an dem der Nahtmechanismus angebracht ist, wobei die Klammermagazinbaugruppe (3) ein Klammermagazin (32) zur Aufnahme von Klammern (31) und die Ambossbaugruppe (2) einen zum Klammermagazin (32) passenden Amboss (21) umfasst, und wobei die Übertragungsbaugruppe (1) mit der Ambossbaugruppe (2) verbunden ist und die Ambossbaugruppe (2) antreibt, um sich relativ zur Klammermagazinbaugruppe (3) zu bewegen;
eine Klammer-Schiebebaugruppe (4) zum Schieben der Klammern (31) in Richtung der Ambossbaugruppe (2), wobei die Klammer-Schiebebaugruppe (4) eine erste Gewindestange (41), die an der Klammermagazinbaugruppe (3) vorgesehen ist, und eine Auslösemutter (42) umfasst, die auf die erste Gewindestange (41) aufgeschoben ist, wobei eine Achsrichtung der ersten Gewindestange (41) und der Auslösemutter (42) als erste Achsrichtung definiert ist; und
eine Antriebsbaugruppe (5), die mit der Übertragungsbaugruppe (1) verbunden ist, um die Übertragungsbaugruppe (1) anzutreiben, damit sie sich bewegt; wobei
das chirurgische Instrument ferner eine an der Ambossbaugruppe (2) vorgesehene Auslösebaugruppe (6) umfasst, wobei die Auslösebaugruppe (6) mit der ersten Gewindestange (41) verbunden ist und die erste Gewindestange (41) in Drehung versetzt, so dass sich die Auslösemutter (42) relativ zur ersten Gewindestange (41) in der ersten Achsrichtung bewegt; **dadurch gekennzeichnet, dass**
die Auslösebaugruppe (6) einen Auslösemotor (61), eine am Auslösemotor (61) vorgesehene Auslöse-Eingangswelle (62) und eine Richtungsänderungs-Übertragungsvorrichtung (63) umfasst, die jeweils mit der Auslöse-Eingangswelle (62) und der ersten Gewindestange (41) verbunden und zwischen diesen angeordnet ist; und
wenn der Nahtmechanismus geschlossen ist, der Auslösemotor (61) die erste Gewindestange (41) über die Richtungsänderungs-Übertragungsvorrichtung (63) in Drehung versetzt;
wobei die Übertragungsbaugruppe (1) eine Übertragungsstange (11) umfasst, wobei ein Ende der Übertragungsstange (11) mit der Antriebsbaugruppe (5) verbunden ist und sich unter der Wirkung der Antriebsbaugruppe (5) dreht, während das andere Ende der Übertragungsstange (11) mit einem Amboss (21) verbunden ist, der in der Ambossbaugruppe (2) enthalten ist, wobei eine Achsrichtung der Übertragungsstange **(11)** als zweite Achsrichtung definiert ist und die erste Achsrichtung senkrecht zur zweiten Achsrichtung steht.

2. Chirurgisches Instrument nach Anspruch 1, wobei die Richtungsänderungs-Übertragungsvorrichtung (63) ein erstes Kegelrad (631), das auf die Auslöse-Eingangswelle (62) aufgeschoben ist, und ein zweites Kegelrad (632) umfasst, das auf die erste Gewindestange (41) aufgeschoben ist, wobei das erste Kegelrad (631) und das zweite Kegelrad (632) miteinander in Eingriff stehen.

3. Chirurgisches Instrument nach Anspruch 2, wobei
die Übertragungsstange (11) eine zweite Gewindestange (11) ist und eine Gewindehülse (12) auf die zweite Gewindestange (11) aufgeschoben ist;
die Antriebsbaugruppe (5) die zweite Gewindestange (11) in Drehung versetzt, so dass sich die Gewindehülse (12) relativ zur zweiten Gewindestange **(11)** in der zweiten Achsrichtung bewegt.

4. Chirurgisches Instrument nach Anspruch 3, wobei
die Übertragungsbaugruppe (1) ferner eine Verschlussbaugruppe (13) umfasst, die auf die zweite Gewindestange (11) aufgeschoben ist, wobei die Verschlussbaugruppe (13) eine erste Verschlussstange (131) und eine zweite Verschlussstange (132) umfasst und die Gewindehülse (12) fest mit der ersten Verschlussstange (131) und der zweiten Verschlussstange (132) verbunden ist, wobei die Gewindehülse (12) zwischen der ersten Verschlussstange (131) und der zweiten Verschlussstange (132) vorgesehen ist;
die erste Verschlussstange (131) mit der Ambossbaugruppe (2) verbunden ist, während die zweite Verschlussstange (132) näher an der Antriebsbaugruppe (5) als die erste Verschlussstange (131) vorgesehen ist;
wenn die Antriebsbaugruppe (5) die zweite Gewindestange (11) in Drehung versetzt, sich die Gewindehülse (12) relativ zur zweiten Gewindestange (11) in der zweiten Achsrichtung bewegt, wodurch die erste Verschlussstange (131) und die zweite Verschlussstange (132) in der zweiten Achsrichtung bewegt werden.

5. Chirurgisches Instrument nach Anspruch 4, wobei
das chirurgische Instrument ferner einen als Anschlag dienenden Sicherheitsschalter (7) umfasst, wobei der Sicherheitsschalter (7) an der Verschlussbaugruppe (13) vorgesehen ist und einen Schaltknopf (71) und eine Schaltwelle umfasst, wobei die Schaltwelle mit dem Schaltknopf (71) verbunden ist, und wobei die zweite Verschlussstange (132) mit einem Durchgangsloch (72) versehen ist, das an die Schaltwelle angepasst ist; und
wenn der Nahtmechanismus geschlossen ist, der Schaltknopf (71) umgelegt und die Schaltwelle in das Durchgangsloch (72) eingeführt wird, um die Verschlussbaugruppe (13) zu verriegeln.

6. Chirurgisches Instrument nach Anspruch 1, wobei
die Ambossbaugruppe ferner einen oberen Backenarm (34) und einen unteren Backenarm (35) umfasst, wobei der obere Backenarm (34) an einer Außenseite des Klammermagazins (32) vorgesehen und mit einem Schlitzloch zur Aufnahme der ersten Gewindestange und der Auslösemutter versehen ist, wobei ein erstes Lager zur Befestigung der ersten Gewindestange in dem Schlitzloch vorgesehen ist;
der untere Backenarm (35) mit einer Hohlkammer (351) zur Aufnahme des Auslösemotors (61) und der Auslöse-Eingangswelle (62) der Auslösebaugruppe (6) versehen ist, wobei die Auslösebaugruppe (6) mechanisch in der Hohlkammer (351) befestigt ist.

7. Chirurgisches Instrument nach Anspruch 1, wobei das chirurgische Instrument ferner einen Griff (100) umfasst, die Übertragungsstange (11) durch ein zweites Lager am Griff (100) befestigt ist, die Antriebsbaugruppe (5) im Griff (100) vorgesehen ist und die Antriebsbaugruppe (5) einen Antriebsmotor, eine mit dem Antriebsmotor verbundene Eingangswelle (51) und ein jeweils mit der Eingangswelle (51) und der Übertragungsstange (11) verbundenes Übertragungselement (52) umfasst.

8. Chirurgisches Instrument nach Anspruch 7, wobei das chirurgische Instrument ferner einen Induktionsschalter (200) zum Erfassen einer Position der Klammermagazinbaugruppe (3) umfasst, wobei der Induktionsschalter (200) im Griff (100) vorgesehen ist.

9. Chirurgisches Instrument nach Anspruch 1, umfassend ferner einen linearen chirurgischen Klammernahtapparat.

10. Chirurgisches Instrument nach Anspruch 3, wobei
die zweite Gewindestange **(11)** nur an einem mittleren Abschnitt der zweiten Gewindestange **(11)** mit einem Gewinde versehen ist; und
wenn der Nahtmechanismus geschlossen ist, sich die Gewindehülse (12) an einem distalen Ende des Gewindes der zweiten Gewindestange **(11)** befindet.

11. Chirurgisches Instrument nach Anspruch 6, wobei eine untere Gewebepositionierungsnadel (343) am oberen Backenarm (34) befestigt ist und in die Ambossbaugruppe (2) eingeführt wird, wenn der Nahtmechanismus geschlossen ist.

12. Chirurgisches Instrument nach Anspruch 7, wobei das Übertragungselement (52) ein erstes Kegelrad (521), das auf die Eingangswelle (51) aufgeschoben ist, und ein zweites Kegelrad (522) umfasst, das auf die Übertragungsstange (11) aufgeschoben ist, wobei das erste Kegelrad (521) mit dem zweiten Kegelrad (522) in Eingriff steht, um eine Drehung der Eingangswelle (51) in Bezug auf die erste Achsrichtung in eine Drehung der Übertragungsstange (11) in Bezug auf die zweite Achsrichtung umzuwandeln.

## Revendications

1. Instrument chirurgical, comprenant :
un mécanisme de suture comprenant un ensemble d'enclume (2) et un ensemble de cartouche d'agrafe (3),
un ensemble de transmission (1), dans lequel l'ensemble de transmission (1) est fourni d'une partie de montage (101), le mécanisme de suture est monté sur la partie de montage (101), l'ensemble de cartouche d'agrafe (3) comprend une cartouche d'agrafe (32) pour accueillir des agrafes (31), l'ensemble d'enclume (2) comprend une enclume (21) adaptée à la cartouche d'agrafe (32), et l'ensemble de transmission (1) est relié à l'ensemble d'enclume (2) et entraîne l'ensemble d'enclume (2) à se déplacer par rapport à l'ensemble de cartouche d'agrafe (3) ;
un ensemble de poussée d'agrafe (4) configuré pour pousser les agrafes (31) à se déplacer vers l'ensemble d'enclume (2), dans lequel l'ensemble de poussée d'agrafe (4) comprend une première tige filetée (41) prévue sur l'ensemble de cartouche d'agrafe (3) et un écrou de tir (42) manchonné sur la première tige filetée (41), une direction daxe de la première tige filetée (41) et de l'écrou de tir (42) est définie comme une première direction d'axe ; et
un ensemble d'entraînement (5), relié à l'ensemble de transmission (1) pour entraîner l'ensemble de transmission (1) à se déplacer ; dans lequel
l'instrument chirurgical comprend en outre un ensemble de tir (6) disposé sur l'ensemble d'enclume (2), l'ensemble de tir (6) est relié à la première tige filetée (41) et entraîne la première tige filetée (41) à tourner, de sorte que l'écrou de tir (42) se déplace par rapport à la première tige filetée (41) dans la première direction d'axe ; **caractérisé en ce que**,
l'ensemble de tir (6) comprend un moteur de tir (61), un arbre d'entrée de tir (62) prévu sur le moteur de tir (61), et un dispositif de transmission de changement de direction (63) respectivement relié à et entre l'arbre d'entrée de tir (62) et la première tige filetée (41) ; et
si le mécanisme de suture est fermé, le moteur de tir (61) entraîne la première tige filetée (41) à tourner via le dispositif de transmission de changement de direction (63) ;
dans lequel l'ensemble de transmission (1) comprend une tige de transmission (11), une extrémité de la tige de transmission (11) est reliée à l'ensemble d'entraînement (5) et tourne par une action de l'ensemble d'entraînement (5), et l'autre extrémité de la tige de transmission (11) est reliée à une enclume (21) incluse dans l'ensemble d'enclume (2), une direction d'axe de la tige de transmission (11) est définie comme une seconde direction d'axe, et la première direction d'axe est perpendiculaire à la seconde direction d'axe.

2. Instrument chirurgical selon la revendication 1, dans lequel le dispositif de transmission de changement de direction (63) comprend un premier engrenage conique (631) manchonné sur l'arbre d'entrée de tir (62) et un second engrenage conique (632) manchonné sur la première tige filetée (41), et le premier engrenage conique (631) et le second engrenage conique (632) sont engagés l'un avec l'autre.

3. Instrument chirurgical selon la revendication 2, dans lequel
la tige de transmission (11) est une seconde tige filetée (11), et un manchon fileté (12) est manchonné sur la seconde tige filetée (11) ;
l'ensemble d'entraînement (5) entraîne la seconde tige filetée (11) à tourner, de sorte que le manchon fileté (12) se déplace par rapport à la seconde tige filetée (11) dans la seconde direction d'axe.

4. Instrument chirurgical selon la revendication 3, dans lequel
l'ensemble de transmission (1) comprend en outre un ensemble de fermeture (13) manchonné sur la seconde tige filetée (11), l'ensemble de fermeture (13) comprend une première tige de fermeture (131) et une seconde tige de fermeture (132), le manchon fileté (12) est relié de manière fixée à la première tige de fermeture (131) et à la seconde tige de fermeture (132), le manchon fileté (12) est prévu entre la première tige de fermeture (131) et la seconde tige de fermeture (132) ;
la première tige de fermeture (131) est reliée à l'ensemble d'enclume (2), la seconde tige de fermeture (132) est prévue plus près de l'ensemble d'entraînement (5) que la première tige de fermeture (131) ;
si l'ensemble d'entraînement (5) entraîne la seconde tige filetée (11) à tourner, le manchon fileté (12) se déplace par rapport à la seconde tige filetée (11) dans la seconde direction d'axe, entraînant ainsi la première tige de fermeture (131) et la seconde tige de fermeture (132) à se déplacer dans la seconde direction d'axe.

5. Instrument chirurgical selon la revendication 4, dans lequel
l'instrument chirurgical comprend en outre un interrupteur de sécurité (7) servant d'un stoppeur, l'interrupteur de sécurité (7) est prévu sur l'ensemble de fermeture (13), l'interrupteur de sécurité (7) comprend un bouton de commutation (71) et un arbre de commutation, l'arbre de commutation est relié au bouton de commutation (71), la seconde tige de fermeture (132) est équipée d'un trou traversant (72) adapté à l'arbre de commutation ; et
si le mécanisme de suture est fermé, le bouton de commutation (71) est basculé, l'arbre de commutation est inséré à l'intérieur du trou traversant (72) pour verrouiller l'ensemble de fermeture (13).

6. Instrument chirurgical selon la revendication 1, dans lequel
l'ensemble d'enclume comprend en outre un bras de mâchoire supérieure (34) et un bras de mâchoire inférieure (35), le bras de mâchoire supérieure (34) est prévu sur une face extérieure de la cartouche d'agrafe (32), le bras de mâchoire supérieure (34) est équipé d'un trou de créneau pour accueillir la première tige filetée et l'écrou de tir, et un premier palier pour fixer la première tige filetée est prévu dans le trou de créneau ;
le bras de mâchoire inférieure (35) est équipé d'une chambre creuse (351) pour accueillir le moteur de tir (61) et l'arbre d'entrée de tir (62) de l'ensemble de tir (6), et l'ensemble de tir (6) est fixé mécaniquement dans la chambre creuse (351).

7. Instrument chirurgical selon la revendication 1, dans lequel l'instrument chirurgical comprend en outre une poignée (100), la tige de transmission (11) est fixée à la poignée (100) par un second palier, l'ensemble d'entraînement (5) est prévu dans la poignée (100), et l'ensemble d'entraînement (5) comprend un moteur d'entraînement, un arbre d'entrée (51) relié au moteur d'entraînement, et un membre de transmission (52) relié respectivement à l'arbre d'entrée (51) et à la tige de transmission (11).

8. Instrument chirurgical selon la revendication 7, dans lequel l'instrument chirurgical comprend un commutateur d'induction (200) pour détecter une position de l'ensemble de cartouche d'agrafe (3), et le commutateur d'induction (200) est prévu dans la poignée (100).

9. Instrument chirurgical selon la revendication 1, comprenant en outre une agrafeuse chirurgicale linéaire.

10. Instrument chirurgical selon la revendication 3, dans lequel
la seconde tige filetée (11) est équipée de filets seulement à une partie médiane de la seconde tige filetée (11) ; et
si le mécanisme de suture est fermé, le manchon fileté (12) est situé sur une extrémité distale des filets de la seconde tige filetée (11).

11. Instrument chirurgical selon la revendication 6, dans lequel une aiguille de positionnement des tissus inférieurs (343) est fixée sur le bras de mâchoire supérieure (34), et l'aiguille de positionnement des tissus inférieurs (343) est insérée à l'intérieur de l'ensemble d'enclume (2) si le mécanisme de suture est fermé.

12. Instrument chirurgical selon la revendication 7, dans lequel le membre de transmission (52) comprend un premier engrenage conique (521) manchonné sur l'arbre d'entrée (51) et un second engrenage conique (522) manchonné sur la tige de transmission (11), le premier engrenage conique (521) est engagé avec le second engrenage conique (522) afin de transformer une rotation de l'arbre d'entrée (51) par rapport à la première direction d'axe en une rotation de la tige de transmission (11) par rapport à la seconde direction d'axe.
